# EUROPEAN PATENT APPLICATION

(11) **EP 4 306 089 A1**
(43) Date of publication of application: **17.01.2024**
(21) Application number: 22184034.1
(22) Date of filing: 11.07.2022
(51) Int. Cl.: A61F 13/02, A61F 13/00

(54) **A WOUND DRESSING AND A METHOD OF MANUFACTURING SAME**

(71) Applicant: Pharmaplast SAE, Alexandria 23512 (EG)
(72) Inventor: RAFAT, Rania, 23512 Alexandria (EG)
(74) Representative: Holme Patent A/S

(57) **Abstract**

A wound dressing (1) comprising a foam layer (2), which is at least partly penetrated by a plurality of spaced apart slits (8,10,11,12,13,14,14a). The foam layer (2) has an adhesive layer (5) on a wound-contacting face (6), and a breathable film layer (3) on the free face (4) opposite the wound-contacting face (6).

## Description

The present invention relates to a wound dressing comprising a foam layer.

Removal of exudate is of the outmost priority when managing high exudate wounds. Advanced wound dressings draw the exudate up into the dressing and attempt to store, optionally lock, said exudate inside the wound dressing.

Most such foam dressings for exudate wounds thus handle fluid by absorbing it and/or allowing it to evaporate. The fluid enters the dressing materials and is drawn into the foam by capillary action or "wicking". Some foam dressings for exudate wounds, such as alginate and hydrofiber dressings, take up fluid to form a gel. So foam dressings are highly absorbent dressings. They absorb wound exudate, prevent strike-through and reduce the time between dressing changes. However, for some wounds exudate levels may exceed the absorbency capacity of the conventional foam dressing. A downside of conventional foam dressings is that they are difficult to dress and to fit closely to the contours of the body to perform effectively.

It is a main aspect of the present invention to provide a simple wound dressing for exudate wounds that has at least the same absorbency capacity as conventional foam dressings, preferably higher absorbency capacity.

It is a further aspect of the present invention to provide a wound dressing for exudate wounds that has improved ability to conform to the contours of the body around the exudate wound.

It is a further aspect of the present invention to provide a wound dressing for exudate wounds that has one or more of the further properties of preventing leakage between dressing changes, preventing strikethrough, provides protection from excoriation and maceration, stays intact, can be left in place for long duration, minimizes trauma and pain on removal, is comfortable to wear, and last but not least is cost-effective.

The novel and unique features whereby these and other aspects are achieved according to the present invention consists in that the foam layer is at least partly penetrated by a plurality of spaced apart slits.

In the context of the present invention the term "slit" means a narrow incision made by a sharp-edged tool or object, e.g. by using a rotary blade cutter having multiple cutting blades arranged to cut, stamp or punch slits in the desired slit pattern for the foam layer of the wound dressing according to the present invention. The pattern of slits may also be provided by e.g. ultrasonic tools or lasers.

The entire blank area and blank volume is available for absorbing exudate. Furthermore, the plurality of penetrating slits opens the cells of the foam structure at the cut faces of said slits, to thereby further facilitate fast wicking of exudate into the foam layer, not only mainly perpendicular to the plane of the foam layer, as the slits provide large opposite faces for exudate to wick along said plane.

Yet a huge advantage is that the plurality of slits makes the wound dressing much more conformable to the area around the wound than known wound dressings. When the foam layer is bending the slits will actually open to some extent and provide an additional pathway for additional evaporation similar to the conventional through-holes.

As the plurality of slits are arranged spaced apart from each other the foam layer retains its structural integrity and does not get apart even when saturated.

Due to the highly improved conformability, and due to retaining its structural integrity, the foam layer of the present invention may be preferred for many different kinds of wound dressings and wound pads, including for other wound dressing than foam dressings for exudate wounds.

The term "foam" should be construed in its broadest sense. Foam materials can be selected to be flexible, compressible, non-compressible and have more or less memory shape properties that allows the foam to restore its original shape after compression, e.g. in case of accidentally being hit, or if the user has slept on the wound dressing. Suitable foams for the foam layer include, but are not limited to, any hydrophilic open cell foams known to the skilled person. Such hydrophilic open cell foams can e.g. be polyurethane (PU) foam, e.g. those described in international patent application no. WO2016/044512. Polyurethane (PU) foam has a high moisture transfer rate that will allow water vapor to evaporate from the face of the dressing opposite the wound-contacting face.

In some embodiments the foam layer may have an adhesive layer on the wound-contacting face so that the foam layer can stay put on the wound. Such an adhesive layer may cover the entire wound-contacting face, a part of the wound-contacting face, e.g. the perimeter of the wound-contacting face, or be provided as distributed spots on the wound-contacting face.

In some embodiments the foam layer may have a breathable film layer on the free face opposite the wound contacting face, whereby the foam layer becomes sandwiched between the adhesive layer and the breathable film layer.

The design of a slit of the plurality of spaced apart slits may be straight, thus extends in one direction only, without a curve or bend. In an alternative design such a slit may follow an open curve. In the context of the present invention the term "open curve" means a bend curvature where the beginning point and end point are different. The "open curve" may be made of one or more of line segments and thus have two free, not connected, endpoints.

In yet an alternative embodiment the plurality of spaced apart slits may comprise combinations of different slit design configurations.

Optionally the slits of the plurality of slits may extend through the entire thickness of the foam layer, whereby the foam layer becomes fully penetrated by the slits of the plurality of slits. In alternative embodiments the slit design configuration may comprise some slits that partly penetrate the foam layer, and some slits that fully penetrate the foam layer.

A design configuration of a slit of the plurality of spaced apart slits may e.g. be L-shaped, V-shaped, U-shaped, S-shaped, Z-shaped, E-shaped, wavy, or C-shaped, or combinations thereof. Within the scope of the present invention the plurality of spaced apart slits may be provided in any imaginable orientation and pattern. If e.g. two adjacent L-shaped slits are arranged to delimit a square-like area or a rectangular-like area, each such L-shaped slit provides a free flap in the angle between the legs of the L-shape if the L-shaped slit penetrate the foam layer in full. If the foam layer is only partly penetrated by the slit such flap is not fully free, but set sufficiently free at the wound-contacting face for the slit to bend open. Opposite flaps are joined due to the distance between the opposite free ends of the opposite L-shaped slits. In embodiments wherein the slits do not penetrate the foam layer in full, the number and density of slits can be higher than if the slits do penetrate the entire thickness of the foam layer, as the face opposite the wound-contacting face remains coherent.

Line segments of the "open curve" may follow in extension of each other via an acute angle, e.g. if a slit is V-shaped, or via an obtuse angle, e.g. if a slit has an open V-shape, or via a smooth bend, e.g. if a slit is U-shaped or C-shaped. In case a slit is L-shaped the angle between its legs will be substantially right.

The adhesive layer may advantageously be a silicone pressure sensitive adhesive (PSA) layer. The PSA layer can be applied using various different methods including, but not limited to spray coating and subsequent curing, or by transfer coating applied to the foam layer. The PSA layer may be applied as a gel.

Within the context of the present invention the term "pressure sensitive adhesive (PSA)" means materials having an aggressive and permanent tack, the ability to adhere with no more than finger pressure, the ability to adhere without activation by any energy source, sufficient ability to hold onto the intended adherend, and preferably sufficient cohesive strength to be removed cleanly from the adherend.

The breathable film layer may advantageously be a polyurethane film. Breathable polyurethane films perform well in lamination and create a liquid barrier for external moisture while allowing enclosed water to evaporate.

The foam layer may be a polyurethane foam layer. A polyurethane foam layer is inexpensive, highly flexible and easy to cut into to make slits without the foam is compressed and/or deforms. Further such a polyurethane foam layer is soft, highly absorbent and reduces pressure over the wound area.

The wound dressing of the present invention may comprise a release liner that covers and protects the adhesive layer, so that the adhesive properties can be preserved during storage of the wound dressing. The release liner on the adhesive layer may be a two part release liner.

An exudate wound dressing needs to be changed sometimes. However due to the large amount of foam material in the close vicinity of the wound, and because the incised slits allows the exudate to easily pass through the adhesive layer and spread well inside the entire foam layer, thereby fast directing the exudate away from the exudate wound, absorption may be faster compared to adhesive layers without slits.

A depth of a slit of the plurality of spaced apart slits may conveniently be an incision about 2/3 of the thickness of the foam layer into said foam layer. Optionally the depth of a slit may correspond to the thickness of the foam layer. Yet an option is that the depth of a slit may correspond so the full thickness of both the adhesive layer and the foam layer, thus the slits penetrates the adhesive layer and more or less of the foam layer.

The present invention further relates to a method of manufacturing a wound dressing material, especially a wound dressing material for manufacturing the above-described wound dressing.

The method comprises the steps of
a) providing a foam layer material,
b) providing an adhesive,
c) optionally providing a breathable film layer material,
d) applying adhesive to one face of the foam layer material to obtain an adhesive foam layer material having an adhesive face,
e) providing from the adhesive face a plurality of spaced apart slits at least partly into the adhesive foam layer material produced in step d), and
f) optionally applying the breathable film layer material to the non-adhesive face of the adhesive foam layer material.

By means of the above method a wound dressing material can be produced as a laminate having two or three layers: An adhesive layer applied to a foam layer and in common having a plurality of spaced apart slits, and optionally a breathable film layer on the opposite face, thus on the non-adhesive face of the foam layer.

Step f) may be performed before step e), in which case attention may in some embodiments be paid not to penetrate the breathable film layer when the plurality of slits are made through the adhesive face into the foam layer. To that aspect the plurality of slits may be made in step e) by kiss-cutting from the adhesive face of the wound dressing material.

The wound dressing material may be manufactured as a long lengths stored in rolls. The wound dressings of the present invention can then be die cut from said wound dressing material, e.g. using a rotary die cutter, optionally after a release liner has been applied.

In step e) the plurality of spaced apart slits can be made without removing material from the foam layer, thereby allowing the entire foam layer to be exposed to exudate, and thus all of the foam layer can absorb exudate. By providing penetrating slits instead of punched-out through-holes all foam layer material, or adhesive foam layer material obtained in step d), remains present in the wound dressing material and the plurality of spaced apart slits promotes wicking away from the wound into the wound dressing into the foam layer towards the breathable film layer.

The invention will now be described in further details with reference to the drawing, in which
Fig. 1 is a perspective exploded view of a first embodiment of a wound dressing according to the present invention, seen from the foam layer, and having a first design configuration of slits,
Fig. 2 shows the same in assembled state,
Fig. 3 shows, in an enlarged scale view, a fragment of the first design configuration of slits, seen from the adhesive face of the foam layer seen in fig. 1,
Fig. 4 is a perspective exploded view of the embodiment seen in figs. 1, but without the breathable film layer,
Fig. 5 shows the same in assembled state,
Fig. 6 shows, in an enlarged scale view, a fragment of a second design configuration of slits, seen from the adhesive face of the foam layer,
Fig. 7 shows, in an enlarged scale view, a fragment of a third design configuration of slits, seen from the adhesive face of the foam layer,
Fig. 8 shows, in an enlarged scale view, a fragment of a fourth design configuration of slits, seen from the adhesive face of the foam layer,
Fig. 9 shows, in an enlarged scale view, a fragment of a fifth design configuration of slits, seen from the adhesive face of the foam layer,
Fig. 10 shows, in an enlarged scale view, a fragment of a sixth design configuration of slits, seen from the adhesive face of the foam layer,
Fig. 11 shows, in an enlarged scale view, a fragment of a seventh design configuration of slits, seen from the adhesive face of the foam layer,
Fig. 12 shows, in an enlarged scale view, a fragment of an eight design configuration of slits, seen from the adhesive face of the foam layer,
Fig. 13 shows a photo of a fragment of a length of wound dressing material for the embodiment of a wound dressing seen in figs. 1 - 3, provided with a release liner on the adhesive face, and seen from the adhesive face, and
Fig. 14 shows the same seen from the breathable film layer.

In the drawing the wound dressings of the present invention are shown with exemplary design configurations of slits. This should not be construed as limiting the scope of the present invention as such slit design can have other configurations than shown. Release liners are shown in some figures, however if the foam layer with more or less penetrating slits alone simply is used as non-adhesive padding no release liners are present.

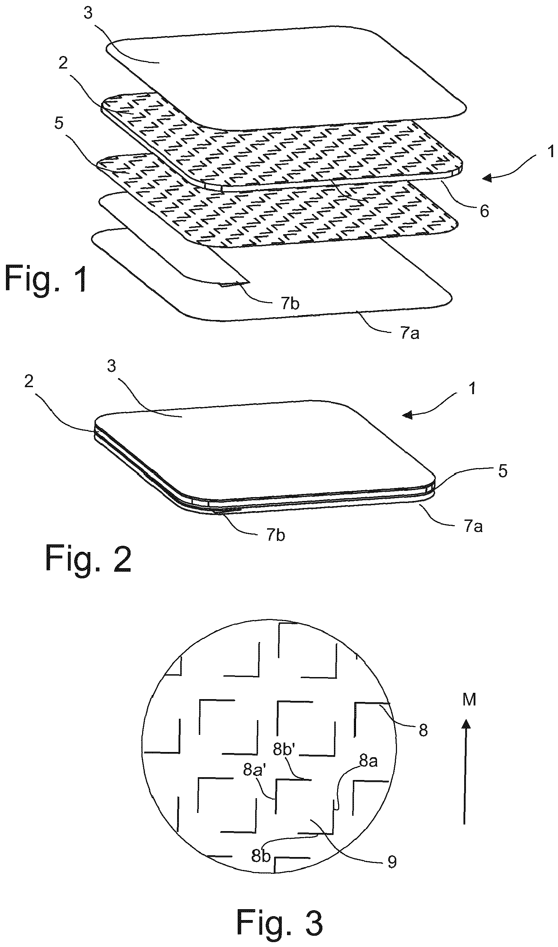

The wound dressing 1 shown in figs. 1 is a laminate comprising a foam layer 2, an exterior breathable film layer 3 on a non-adhesive face 4 of said foam layer 2, and an adhesive layer 5 on the face 6 opposite the non-adhesive face 4, which face 6 thus is the adhesive face 6, and thereby the wound-contacting face, which may be protected by a release liner 7a,7b.

The central foam layer 2 has a plurality of spaced apart slits 8, which in the present embodiment all are L-shaped. The L-shaped slits 8 are arranged in a pattern, grouped in pairs 9. A pair 9 defines a substantially rectangular outline, as seen best in fig. 3, wherein an enlarged scale fragment of the adhesive face 4 of the foam layer 2 seen in fig. 1 is seen in plane view. The angle between the legs 8a,8b of one L-shaped slit 8 faces towards the angle between the legs 8a',8b' of the other L-shaped slit 8 of a pair 9. The angle between the legs 8a,8b;8a',8b' of the respective opposite facing L-shaped slits 8 is substantially right, but could quite as well be both obtuse and acute. The L-shaped slits 8 are cut through or into the foam layer 2 from the adhesive face 6.

The length of the legs 8a,8b;8a',8b' may be the same or different. In fig. 3 arrow M indicates the machine direction for making the plurality of spaced apart slits 8. In relation to the machine direction M the legs 8a are called forward vertical legs, and the legs 8a' are called backwards vertical legs. Seen in relation to the orientation of the slits 8, as viewed in fig. 3, the legs 8b connected to the forward vertical legs 8a are called left horizontal legs, and the legs 8b' connected to the backwards vertical legs 8a' are called right horizontal legs.

In the exemplary embodiment seen in figs. 1 - 3 the length of the legs 8a,8b;8a',8b' is 7 mm, and the depth of a slit 8 is 3 mm for a corresponding combined thickness of 3 mm of the foam layer 2 and the adhesive layer 5. The distance between backwards vertical legs 8a' of neighboring pairs 9 may be 16 mm, the distance between a forward vertical leg 8a and a backwards vertical leg 8a' of a pair 9 may be 11 mm, the distance between a forward vertical leg 8a of one pair 9 and a backwards vertical leg 8a' of a neighboring pair 9 may be 5 mm, the distance between a free end of backwards vertical legs 8a' of subsequent pairs 9, seen in the machine direction M, may be 15-16 mm, and the distance between a right horizontal leg 8b' and a free end of a backwards horizontal leg 8a', seen in the machine direction M, may be about 5 mm. These lengths and dimensions are only examples. They should not be seen as limiting the scope of the present invention. Lengths and distances between legs or segments of a slit 8 and of neighboring slits 8 can be longer or shorter. Variations can be applied by selecting the appropriate slitting machinery.

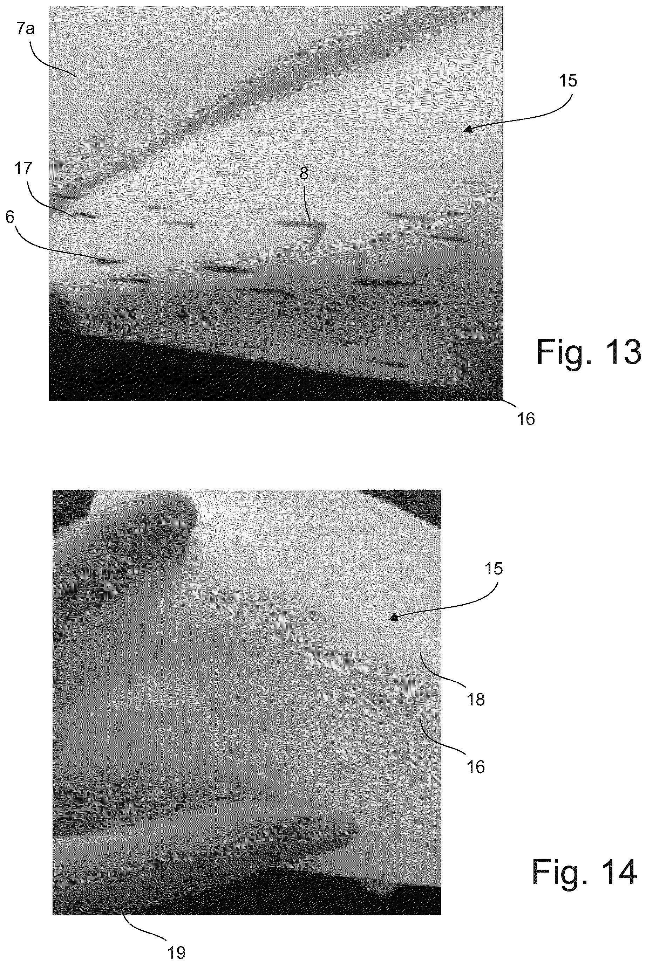

Since the breathable film layer 3 is rather elastic it yields when the foam layer 2 is bending to conform to a given subjacent curvature, whereby the slits 8 open to some extent, as seen in the photos of figs. 14 and 15, to further promote entry of moisture, e.g. from an exudate wound.

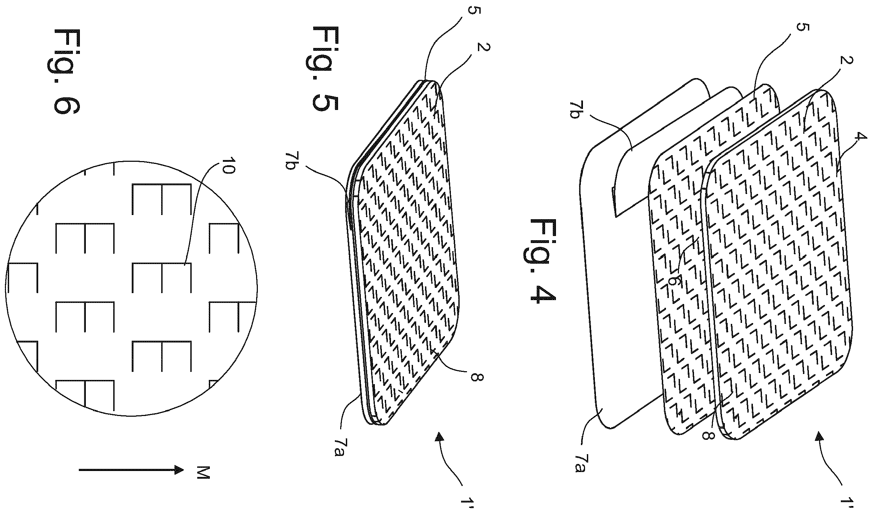

Fig. 4 is a perspective exploded view of a second embodiment 1', which corresponds to the wound dressing 1 seen in figs. 1, but without the breathable film layer 3. Fig. 5 shows the same in assembled state.

Below is briefly mentioned, with reference to a corresponding figure, various design configurations of slits. How dense the slits of the plurality of slits are arranged may vary depending on e.g. the intended purpose of use, and of the size of the wound dressing.

Fig. 6 shows, in an enlarged scale view, a fragment of a second design configuration of slits 10, seen from the adhesive face 4 of the foam layer 2, wherein the slits 10 are E-shaped. The E-shaped slits 10 are arranged in rows, and the rows are off-set each other to arrange an upstream E-shaped slit 10 above the gap between two adjacent E-shaped slits 10 of the forgoing row, when seen in the machine direction M.

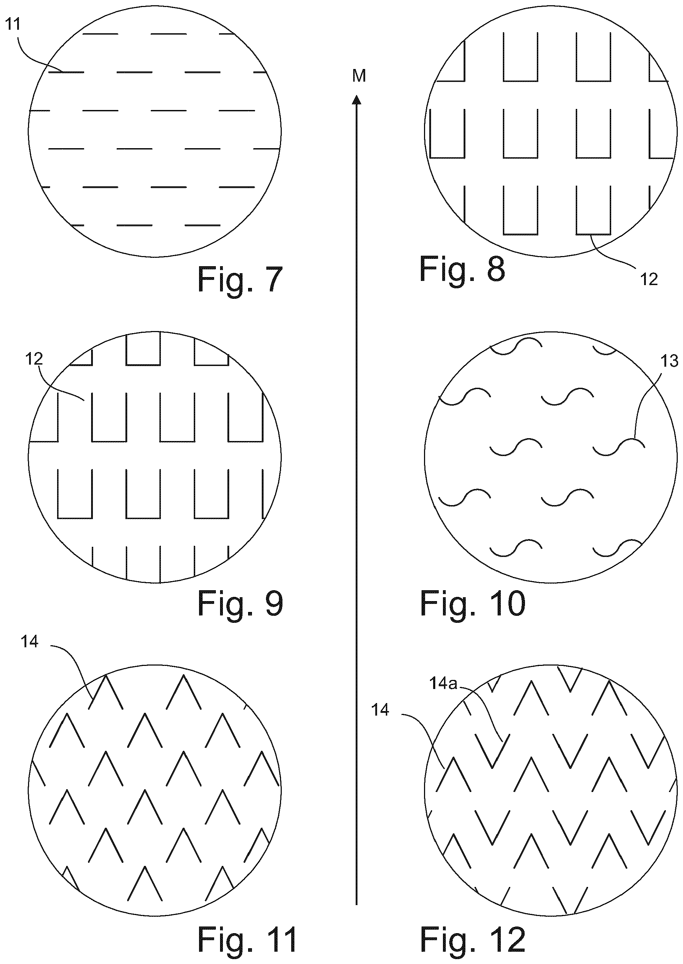

Fig. 7 shows, in an enlarged scale view, a fragment of a third design configuration of slits 11, seen from the adhesive face 4 of the foam layer 2, wherein the slits 11 are straight line segments arranged in rows, and wherein the rows are off-set each other to arrange an upstream straight slit 11 above the gap between two adjacent straight slits 11 of the forgoing row, when seen in the machine direction M. The slits 11 are traverse to the machine direction M but could also be provided with an angle to the machine direction.

Fig. 8 shows, in an enlarged scale view, a fragment of a fourth design configuration of slits 12, seen from the adhesive face 4 of the foam layer 2, wherein the slits 12 have sharp U-shapes, are arranged in rows, and are aligned in the machine direction M.

The embodiment of a fifth design configuration of slits 12 seen in fig. 9 resembles the fourth design configuration of slits 12 seen in fig. 8, but with the difference that the sharp U-shaped slits 12 are arranged in rows but are off-set each other to arrange an upstream sharp U-shaped slit 12 above the gap between two adjacent sharp U-shaped slits 12 of the forgoing row, when seen in the machine direction M.

Fig. 10 shows, in an enlarged scale view, a fragment of a sixth design configuration of slits 13, seen from the adhesive face 4 of the foam layer 2, wherein the slits 13 have lying S-shapes, when seen in the machine direction M. The S-shaped slits 13 are arranged in rows, and wherein the rows are off-set each other to arrange an upstream S-shaped slit 13 above the gap between two adjacent S-shaped slits 13 of the forgoing row.

Fig. 11 shows, in an enlarged scale view, a fragment of a seventh design configuration of slits 14, seen from the adhesive face 4 of the foam layer 2. The slits 14 are arrow-pointed, thus V-shaped, with the legs of the V arranged in an acute angle to each other. The arrow points of the V-shaped slits 14 are directed in the machine direction M. The V-shaped slits 14 are arranged in rows, and wherein the rows are off-set each other to arrange an upstream V-shaped slit 14 above the gap between two adjacent V-shaped slits 14 of the forgoing row.

Fig. 12 shows, in an enlarged scale view, a fragment of an eight design configuration that resembles the seventh design configuration seen in fig. 11. The eight design configuration differs from the seventh design configuration in that every second row of V-shaped slits is turned upside down in relation to the machine direction M, whereby the points of the V-shaped slits of a turned row face opposite the machine direction M. So the eight design configuration has alternating first rows and second rows, having V-shaped slits 14 pointing in the machine direction M and second rows having V-shaped slits 14a pointing opposite the machine direction M, respectively. Further, the first rows are off-set the second rows to arrange a V-shaped slit 14 above the gap between two adjacent V-shaped slits 14a of the second row. This arrangement of V-shaped slits 14,14a allows a large number of slits 14,14a to be provided to the foam layer 2 and the adhesive layer 5 without said foam layer 2 and said adhesive layer 5 loose their structural integrity.

Fig. 13 shows a photo of a fragment of a length of a wound dressing material 15 for the embodiment of a wound dressing 1 seen in figs. 1 -3.

The wound dressing material 15 has a release liner 7a that has been partly removed from the face 6 of the foam layer material 16, e.g. polyurethane foam, which has the adhesive 17, e.g. provided by a PSA gel coating, on a wound-contacting face 6, to obtain the adhesive layer 5. The foam layer material 16 for the foam layer 2 of the wound dressing 1 is seen to be very bendable, and thus conformable, and the slits 8 open when the foam layer 2 / foam layer material 16 is bend.

Fig. 14 shows the same seen from the breathable film material 18, through which the foam layer material 16 is visible. The breathable film material 18 does not stick to the fingers 19, and does not negatively affect conformability due to its inherent elasticity.

## Claims

1. A wound dressing (1) comprising a foam layer (2), **characterised in that** the foam layer (2) is at least partly penetrated by a plurality of spaced apart slits (8, 10, 11, 12, 13, 14, 14a).

2. A wound dressing (1) according to claim 1, **characterised in that** the foam layer (2) has an adhesive layer (5) on a wound-contacting face (6), and optionally the foam layer (2) has a breathable film layer (3) on the free face (4) opposite the wound-contacting face (6).

3. A wound dressing (1) according to any of claims 1 or 2, **characterised in that** a slit (11) of the plurality of spaced apart slits (8,10,11,12,13,14,14a) is straight, or follows an open curve, or the plurality of spaced apart slits (8,10,11,12,13,14,14a) comprises combinations of different slit design configurations, optionally the slits (8,10,11,12,13,14,14a) of the plurality of spaced apart slits (8,10,11,12,13,14,14a) extends through the entire thickness of the foam layer (2).

4. A wound dressing (1) according to any of claims 1, 2 or 3, **characterised in that** a design configuration of a slit (8,10,11,12,13,14,14a) of the plurality of spaced apart slits (8,10,11,12,13,14,14a) are L-shaped, V-shaped, U-shaped, S-shaped, Z-shaped, E-shaped, wavy, C-shaped, or combinations thereof.

5. A wound dressing (1) according to any of the preceding claims 1 - 4, **characterised in that** the wound dressing (1) comprises one or more of
- the adhesive layer (5) is a silicone pressure sensitive adhesive,
- the breathable film layer (3) is a polyurethane film, and
- the foam layer (2) is a polyurethane foam layer.

6. A wound dressing (1) according to any of the preceding claims 2 - 5, **characterised in that** one or both of the adhesive layer (5) and the breathable film layer (3) is covered by a release liner (7a,7b).

7. A wound dressing (1) according to any of the preceding claims 1 - 6, **characterised in that** the wound dressing (1) is an exudate wound dressing (1).

8. A wound dressing (1) according to any of the preceding claims 1 - 7, **characterised in that** a depth of a slit (8,10,11,12,13,14,14a) of the plurality of spaced apart slits (8,10,11,12,13,14,14a) is an incision about 2/3 of the thickness of the foam layer (2) into said foam layer (2), optionally the depth of a slit (8,10,11,12,13,14,14a) corresponds to the thickness of the foam layer (2).

9. A wound dressing (1) according to any of the preceding claims 2 - 7, **characterised in that** the depth of a slit (8,10,11,12,13,14,14a) extends through the adhesive layer (5) through, or into, the foam layer (2); optionally the depth of the slits (8,10,11,12,13,14,14a) corresponds to the combined thickness of the foam layer (2) and the adhesive layer (5).

10. A method of manufacturing a wound dressing material (15), **characterised in** the steps of
a) providing a foam layer material (16),
b) providing an adhesive (17),
c) optionally providing a breathable film layer material (18),
d) applying adhesive (17) to one face of the foam layer material (16) to obtain an adhesive foam layer material having an adhesive face (6),
e) providing from the adhesive face (6) a plurality of spaced apart slits (8,10,11,12,13,14,14a) at least partly into the adhesive foam layer material produced in step d), and
f) optionally applying the breathable film layer material (18) to the non-adhesive face (4) of the adhesive foam layer material.

11. A method of manufacturing a wound dressing material (15) according to claim 10, **characterised in that** step f) is performed before step e).

12. A method of manufacturing a wound dressing material (15) according to any of the preceding claims 10 or 11, **characterised in that** the plurality of spaced apart slits (8,10,11,12,13,14,14a) are made in step e) by kiss-cutting from the adhesive face (6) of the wound dressing material (15) .

13. A method of manufacturing a wound dressing material (15) according to any of the preceding claims 10, 11 or 12, **characterised in that** in step e) the plurality of spaced apart slits (8,10,11,12,13,14,14a) are made without removing material from the foam layer material (2), or from the adhesive foam layer material obtained in step d).

14. A method of manufacturing a wound dressing (1) according to any of the preceding claims 1 - 9 of the wound dressing material (15) according to any of the preceding claims 10 - 13, **characterised in that** the wound dressing (1) is die cut from the wound dressing material (15).

15. A wound dressing material (15) comprising a foam layer material (16) at least partly penetrated by a plurality of spaced apart slits (8,10,11,12,13,14,14a), optionally the foam layer material (16) is penetrated through its entire thickness by the plurality of spaced apart slits (8, 10, 11, 12, 13, 14, 14a).
